(19) Europäisches Patentamt
European Patent Office
Office européen des brevets

(11) Veröffentlichungsnummer: **0 622 424 A1**

(12) **EUROPÄISCHE PATENTANMELDUNG**

(21) Anmeldenummer: **94105981.8**

(22) Anmeldetag: **18.04.94**

(51) Int. Cl.[5]: **C09B 62/03**, C09B 62/09, C09B 62/25, C09B 62/41, C09B 62/513, C09B 31/08, C07C 309/50, D06P 3/24, D06P 3/66

(30) Priorität: **30.04.93 DE 4314300**

(43) Veröffentlichungstag der Anmeldung: **02.11.94 Patentblatt 94/44**

(84) Benannte Vertragsstaaten: **CH DE FR GB LI**

(71) Anmelder: **BAYER AG**
**D-51368 Leverkusen (DE)**

(72) Erfinder: **Kunde, Klaus, Dr.**
**Moltkestrasse 5**
**D-53819 Neunkirchen-Seelscheid (DE)**

(54) **Reaktivfarbstoffe.**

(57) Reaktivfarbstoffe, ein Verfahren zu ihrer Herstellung und ihre Verwendung sowie neue Zwischenprodukte und ein Verfahren zu ihrer Herstellung.

Es werden Reaktivfarbstoffe der Formel (I)

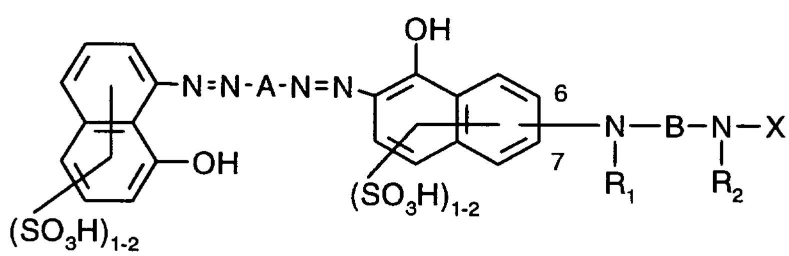

(I)

beschrieben, worin

A einen Rest der Formel

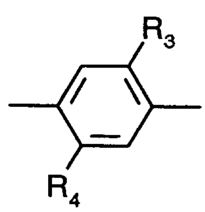

oder

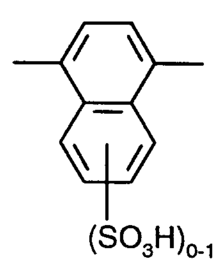

,

B ein Brückenglied bedeutet,

EP 0 622 424 A1

und $R_1$, $R_2$, $R_3$, $R_4$ und X die in der Beschreibung genannten Bedeutungen besitzen.

Die Erfindung betrifft Reaktivfarbstoffe, die in Form der freien Säure der allgemeinen Formel (I) entsprechen,

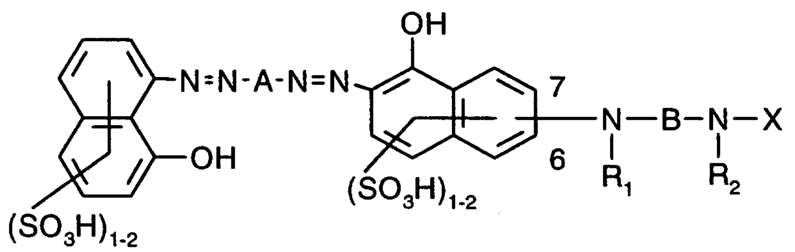
(I)

in der der Rest

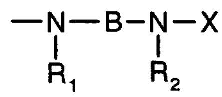

an die 6- oder 7-Position des Naphthalinsystems gebunden ist,
worin

A einen Rest der Formel

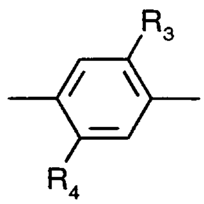
oder
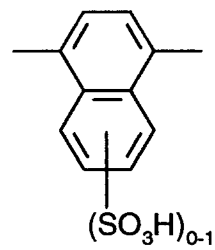

B ein Brückenglied,

$R_1$ und $R_2$ unabhängig voneinander H, gegebenenfalls substituiertes $C_1$-$C_3$-Alkyl, gegebenenfalls substituiertes Phenyl, insbesondere Sulfophenyl bedeuten, wobei als Substituenten beispielsweise OH, $SO_3H$, $CH_3$, $C_2H_5$, $OCH_3$, $OC_2H_5$ oder $OC_3H_7$ in Frage kommen oder

B, $R_1$ und $R_2$ zusammen mit den beiden Stickstoffatomen ein gegebenenfalls substituiertes Piperazin bilden, wobei als Substituenten beispielsweise OH, $SO_3H$, $CH_3$, $C_2H_5$, $OCH_3$, $OC_2H_5$ oder $OC_3H_7$ in Frage kommen,

$R_3$ H, $C_1$-$C_4$-Alkyl, insbesondere $CH_3$, $C_1$-$C_4$-Alkoxy, insbesondere $OCH_3$ oder $OC_2H_5$ oder $SO_3H$,

$R_4$ H, $C_1$-$C_4$-Alkyl, insbesondere $CH_3$, $C_1$-$C_4$-Alkoxy, insbesondere $OCH_3$ oder $OC_2H_5$ sowie $NHCOCH_3$, $NHCONH_2$, $NHCOCH_2OH$ bedeutet und

X ein Reaktivsystem aus der Reihe der 2,3-Dichlorchinoxaline, der Pyrimidine oder der Triazine darstellt,

wobei Reaktivfarbstoffe der Formel (I) ausgenommen sind, worin

B eine sulfogruppenhaltige Phenylengruppe und

X einen 2-Chlor-4-amino-1,3,5-triazinrest oder einen 2-Chlor-4-sulfoanilino-1,3,5-triazinrest bedeutet, die bereits aus DE-A-1 151 625 (US-A-2 993 038) bekannt sind.

Bevorzugt sind Farbstoffe der Formel (I), worin

X für einen Triazinrest steht, der mit einem Amin substituiert ist, welches gegebenenfalls eine Vinylsulfonyl- oder eine unter alkalischen Bedingungen in diese überführbare Gruppe enthält oder der mit einem Diamin substituiert ist, welches gegebenenfalls an dem Aminstickstoff, das nicht mit dem Triazinrest verknüpft ist, mit weiteren Reaktivsystemen

aus der Reihe der Pyrimidine substituiert ist,
und die übrigen Substituenten die obige Bedeutung besitzen;
weiterhin bevorzugt sind Farbstoffe, die in Form ihrer freien Säure der allgemeinen Formel (II) entsprechen,

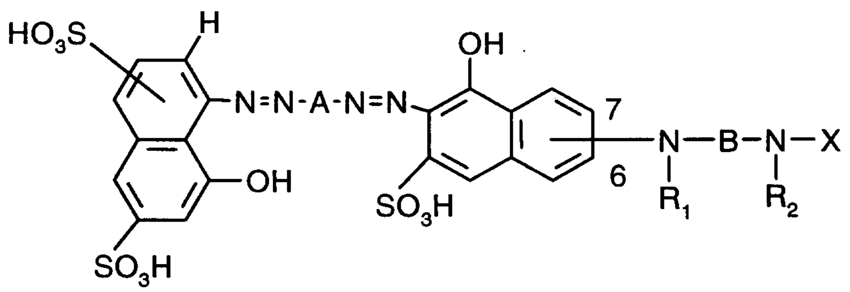 (II)

in der der Rest

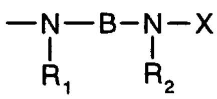

an die 6- oder 7-Position des Naphthalinsystems gebunden ist,
worin

A ein Rest der Formel

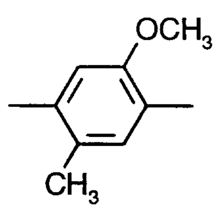 , 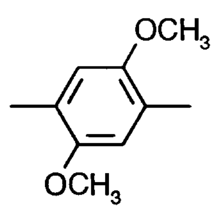 oder 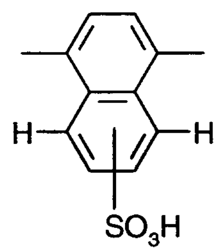 ,

$R_1$ H, $CH_3$ oder $C_2H_4OH$,
$R_2$ H, $CH_3$, $C_2H_4OH$, $C_2H_4SO_3H$, $C_6H_5$ oder $C_6H_4SO_3H$ bedeutet oder
$R_1$ und $R_2$ zusammen mit B und den beiden N-Atomen ein Piperazin bilden,
B geradkettiges oder verzweigtes $C_2$-$C_6$-Alkylen, $C_5$-$C_6$-Cycloalkylen, gegebenenfalls mit einer Sulfogruppe substituiertes Phenylen, einen Rest der Formel $-C_2H_4-O-C_2H_4-$ oder einen Rest der Formel

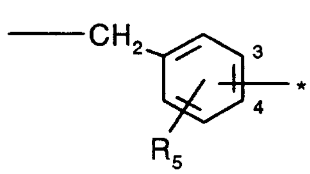

bedeutet, worin die Gruppe

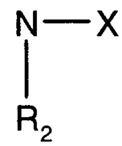

über die mit * markierte Bindung mit der 3- oder 4-Position des Rings verknüpft ist,

$R_5$ insbesondere für H oder $SO_3H$ steht und

X die oben angegebene Bedeutung besitzt,

wobei die oben gemachte Ausnahme auch für die Farbstoffe der Formel (II) gilt.

Besonders bevorzugt sind Farbstoffe, die in Form ihrer freien Säure der allgemeinen Formel (III) entsprechen,

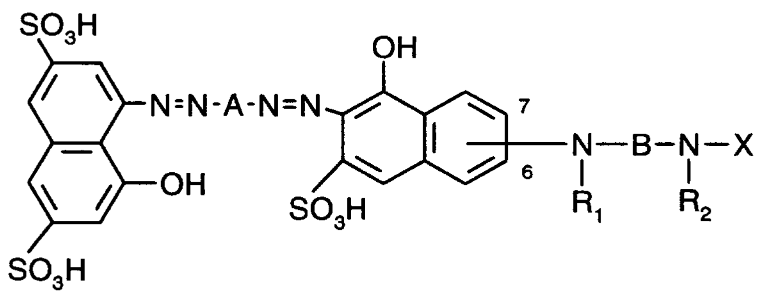 (III)

worin

B $C_2H_4$, $C_3H_6$, $C_4H_8$ oder einen Rest der Formel

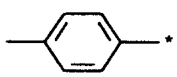 , 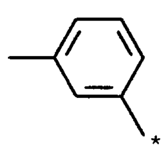 , 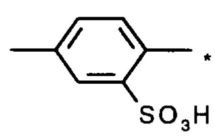 , 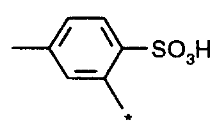 ,

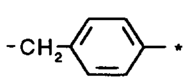 , 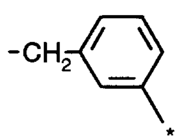 , 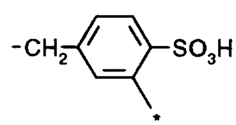 oder 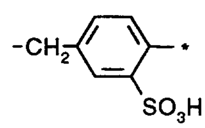

bedeutet,
wobei die Gruppe

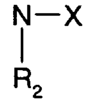

über die mit * markierte Bindung verknüpft ist,

X einen Rest eines Reaktivsystems, insbesondere der allgemeinen Formel

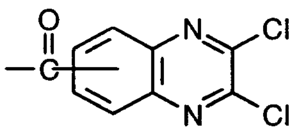 ,

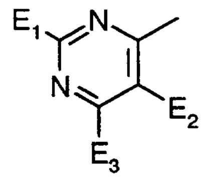 ,

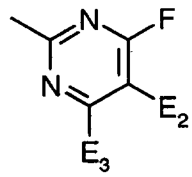 ,

(IV) (Va) (Vb)

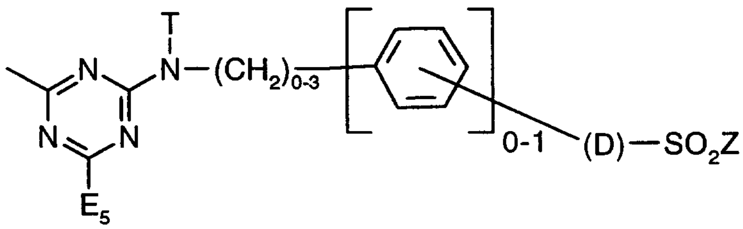

(VI) ,

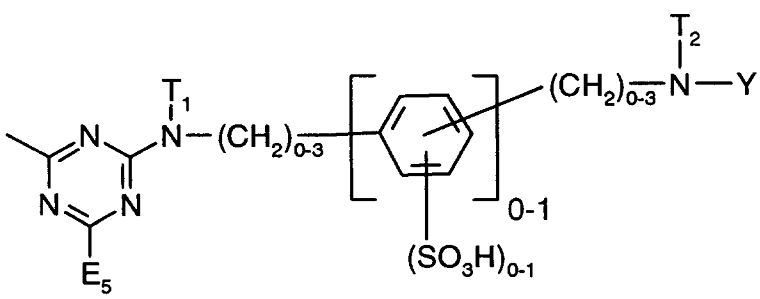

(VII)

oder

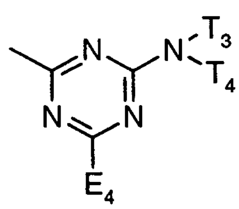

(VIII)

bedeutet,
worin

$E_1$ für H oder F,
$E_2$ für H, Cl oder CN,
$E_3$ für F oder $CH_3$,
$E_4$, $E_5$ für F oder Cl steht,
T, $T_1$ H, gegebenenfalls substituiertes $C_1$-$C_4$-Alkyl, insbesondere $CH_3$ oder $C_2H_5$ sowie $C_2H_4OH$ oder gegebenenfalls substituiertes Phenyl,
$T_2$ H, gegebenenfalls substituiertes $C_1$-$C_4$-Alkyl, insbesondere $CH_3$ oder $C_2H_5$ sowie $C_2H_4OH$,
$T_3$ H, gegebenenfalls substituiertes $C_1$-$C_4$-Alkyl, insbesondere $C_2H_5OH$, gegebenenfalls sulfonsäuregruppenhaltiges Phenyl, beispielsweise $C_6H_4SO_3H$ oder $C_6H_3(SO_3H)_2$,
$T_4$ H, gegebenenfalls substituiertes $C_1$-$C_4$-Alkyl, insbesondere $CH_3$ oder $C_2H_5$ sowie $C_2H_4OH$,

wobei die gegebenenfalls substituierten $C_1$-$C_4$-Alkylreste in der Bedeutung von T, $T_1$, $T_2$, $T_3$ und $T_4$ beispielsweise mit Substituenten wie OH, $OCH_3$, $OC_2H_5$ oder $OC_2H_4OH$ substituiert sein können,

D ein Alkylenrest, der gegebenenfalls durch Sauerstoff unterbrochen sein kann, insbesondere ein Rest der Formel $-(CH_2)_{1-6}$-oder $-C_2H_4-O-C_2H_4-$,

Y ein Rest der Formeln (Va) oder (Vb),

Z $-CH=CH_2$ oder eine unter alkalischen Bedingungen in Vinylsulfonyl überführbare Gruppe, insbesondere $-CH_2CH_2OSO_3H$ oder $-CH_2CH_2Cl$ bedeutet und

A, $R_1$, $R_2$ die oben angegebenen Bedeutungen besitzen.

Ganz besonders bevorzugt sind Reaktivfarbstoffe der Formel (III), worin

B $C_2H_4$, $C_3H_6$, $C_4H_8$ oder einen Rest der Formel

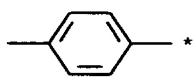 , 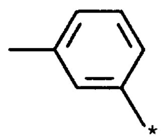 , 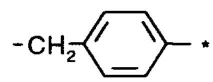 ,

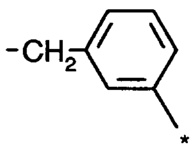 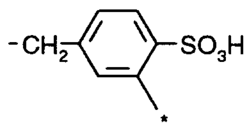 oder 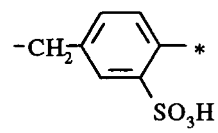 ,

bedeutet, wobei die Gruppe

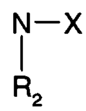

über die mit * markierte Bindung verknüpft ist und A, $R_1$, $R_2$ und X die oben angegebene Bedeutung besitzen.

Weiterer Gegenstand der Erfindung ist ein Verfahren zur Herstellung von Reaktivfarbstoffen der Formel (I), dadurch gekennzeichnet, daß Farbbasen der allgemeinen Formel (IX)

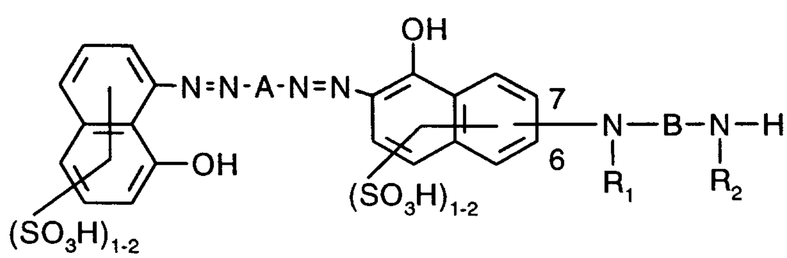 (IX)

worin A, B, $R_1$ und $R_2$ die oben angegebenen Bedeutungen besitzen,
und in der der Rest

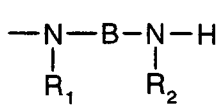

an die 6- oder 7-Position des Naphthalinsystems gebunden ist,
mit Reaktivsystemen der Formel

Hal - X

umgesetzt werden, worin

X die oben angegebene Bedeutung besitzt,

Hal für Halogen, insbesondere F oder Cl steht,

und die oben ausgenommenen Farbstoffe auch für das Verfahren ausgenommen sind.

Als Reaktivsysteme der Pyrimidin-Reihe können z.B. 5-Chlor-2,4,6-trifluorpyrimidin, 5-chlor-4,6-difluorpyrimidin, 5-Cyano-2,4,6-trifluorpyrimidin, 2,4,6-Trifluorpyrimidin, 4,5,6-Trifluorpyrimidin, 5-Chlor-2,4-difluor-6-methylpyrimidin, als solche der Chinoxalin-Reihe 2,3-Dichlorchinoxalin-6-carbonsäurechlorid und als solche der Triazin-Reihe 2,4,6-Trichlor-1,3,5-triazin oder 2,4,6-Trifluor-1,3,5-triazin Verwendung finden.

Die Farbbasen der allgemeinen Formel (IX) können insbesondere dadurch erhalten werden, daß man Diazoniumverbindungen der allgemeinen Formel

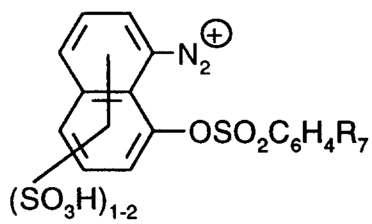

(X)

auf Kupplungskomponenten der allgemeinen Formel

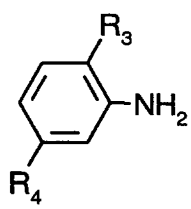

(XIa)

oder

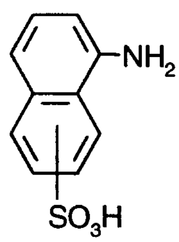

(XIb)

kuppelt, die so erhaltenen Aminoazoverbindungen ihrerseits diazotiert und diese Diazoniumazoverbindungen mit Kupplungskomponenten der allgemeinen Formel

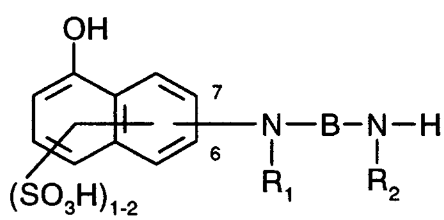

(XII)

kuppelt und das erhaltene Disazoprodukt der Formel (XIII)

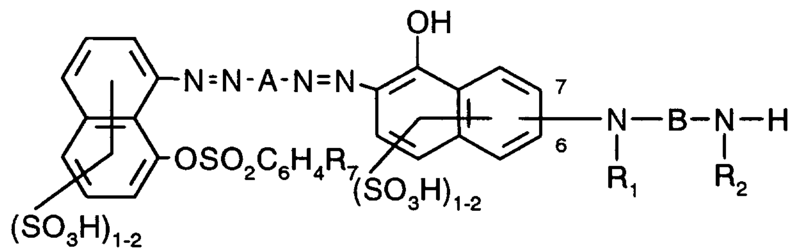

(XIII),

worin

A und B die oben genannte Bedeutung haben und

$R_7$ Wasserstoff, $C_1$-$C_4$-Alkyl oder Halogen bedeutet, verseift.

In einer besonderen Ausführungsform dieses Verfahrens werden Kupplungskomponente der Formel (XII) eingesetzt, worin B, $R_1$ und $R_2$ die unter den neuen Farbbasen der Formel (IX) angegebene Bedeutung haben.

Zum Gegenstand der Erfindung gehören daher auch neue Verbindungen der Formel (XIII) worin A und $R_7$ die oben angegebene Bedeutung besitzen und B, $R_1$ und $R_2$ die unter den neuen Farbbasen der Formel (IX) angegebene Bedeutung haben.

Diazoniumverbindungen der Formel (X) werden vorzugsweise durch Diazotierung der O-Benzolsulfonsäureester von z.B. H-Säure (1-Amino-8-Hydroxy-3,6-naphthalindisulfonsäure), K-Säure (1-Amino-8-Hydroxy-4,6-naphthalindisulfonsäure), 5-Amino-4-hydroxy-2-naphthalinsulfonsäure oder 4-Amino-5-hydroxy-1-naphthalinsulfonsäure erhalten.

Kupplungskomponenten der Formel (XIa) oder (XIb) sind beispielsweise Anilin, 2-Methoxyanilin, m-Toluidin, 2-Methoxy-5-methylanilin, 3-Acetylaminoanilin, 2,5-Dimethoxyanilin, 3-Aminophenylharnstoff, N-3-Aminophenylglykolsäureamid, 4-Acetylamino-2-aminobenzolsulfonsäure, 1-Aminonaphthalin, 5-Amino-2-naphthalinsulfonsäure oder 8-Amino-2-naphthalinsulfonsäure.

Weiterer Gegenstand der Erfindung sind neue Farbbasen der Formel IX, worin A die obengenannte Bedeutung hat,

$R_1$ für H, $CH_3$ oder $C_2H_4OH$,

$R_2$ für H, $CH_3$, $C_2H_4OH$, $C_2H_4SO_3H$, $C_6H_5$ oder $C_6H_4SO_3H$ steht,

B geradkettiges oder verzweigtes $C_2$-$C_6$-Alkylen, $C_5$-$C_6$-Cycloalkylen, einen Rest der Formel $-C_2H_4-O-C_2H_4-$ oder einen Rest der Formel

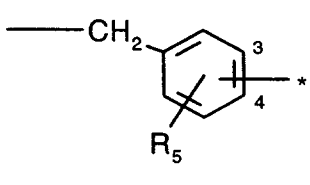

bedeutet, worin die Gruppe

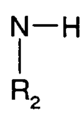

über die mit * markierte Bindung mit der 3- oder 4-Position des Rings verknüpft ist und

$R_5$ für H oder $SO_3H$ steht.

Ebenso Gegenstand der Erfindung sind neue Kupplungskomponenten der Formel (XII), worin

$R_1$ H, $CH_3$ oder $C_2H_4OH$,

$R_2$ H, $CH_3$, $C_2H_4OH$, $C_2H_4SO_3H$, $C_6H_5$ oder $C_6H_4SO_3H$ bedeutet, wobei $R_1$ und $R_2$ vorzugsweise eine unterschiedliche Bedeutung haben,

B geradkettiges oder verzweigtes $C_2$-$C_6$-Alkylen, vorzugsweise $C_3$-$C_6$-Alkylen, $C_5$-$C_6$-Cycloalkylen, ein Rest der Formel $-C_2H_4-O-C_2H_4-$ oder einen Rest der Formel

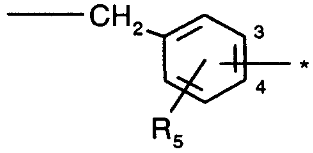

bedeutet, worin die Gruppe

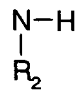

über die mit * markierte Bindung mit der 3- oder 4-Position des Rings verknüpft ist und

$R_5$ für H oder $SO_3H$ steht,

mit der Maßgabe, daß $R_2 \neq$ Wasserstoff, wenn B für Ethylen und $R_1$ für Wasserstoff steht.

In einer weiteren Ausführungsform ist $R_2 \neq$ Wasserstoff, wenn B für Propylen oder Butylen und $R_1$ für Wasserstoff steht.

Diese erfindungsgemäßen Kupplungskomponenten werden vorzugsweise durch Buchererreaktionen der entsprechenden Aminohydroxynaphthalinsulfonsäuren oder Dihydroxynaphthalinsulfonsäuren der allgemeinen Formel

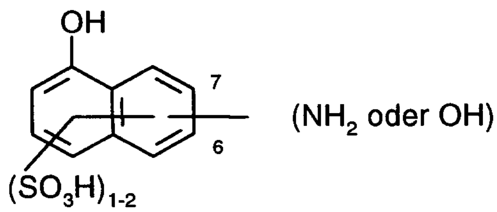

mit Diaminen der allgemeinen Formel

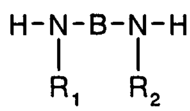 (XIV )

oder Monocarbonsäureamiden der allgemeinen Formel

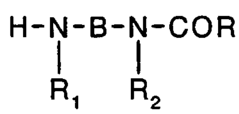 (XIVa )

erhalten, worin

R ein Alkyl- oder Arylrest ist, z.B. $CH_3$, $C_2H_5$, $CH=CH\text{-}COOH$ oder $C_6H_5$ und

B, $R_1$ und $R_2$ die für die erfindungsgemäßen Kupplungskomponenten der Formel (XII) oben angegebene Bedeutung besitzen.

Bei Verwendung der Monoamide wird der Acylrest nach der Buchererreaktion sauer oder alkalisch abgespalten.

Ausgangsverbindungen für die Buchererreaktionen sind z.B. I-Säure (1-Hydroxy-3-sulfo-6-aminonaphthalin), Gammasäure (1-Hydroxy-3-sulfo-7-aminonaphthalin), 4,7-Dihydroxy-2-naphthalinsulfonsäure, 2-Amino-5-hydroxy-1,7-naphthalindisulfonsäure, 3-Amino-5-hydroxy-2,7-naphthalindisulfonsäure einerseits sowie andererseits z.B. 1,2-Diaminoethan, 1,2-Diaminopropan, 1,3-Diaminopropan, 1,4-Diaminobutan, N,N'-Dimethylethylendiamin, 1-(2'-Hydroxyethylamino)-2-aminoethan, 2-(2'-Aminoethylamino)-ethansulfonsäure, 1,2-Diaminocyclopentan, 1,2-Diaminocyclohexan, 1,4-Diaminocyclohexan, 1,3-Diaminobenzol, 1,4-Diaminobenzol, N-(3-Methylaminophenyl)-essigsäureamid, N-(4-Methylaminophenyl)-essigsäureamid, 2,4-Diaminobenzolsulfonsäure, 2,5-Diaminobenzolsulfonsäure, 4-Aminomethylanilin, 3-Aminomethylanilin, 4-Methylaminomethylanilin, 3-Methylaminomethylanilin, N-(4-Aminomethylphenyl)-acetamid, N-(3-Aminomethylphenyl)-acetamid, N-(4-Methylaminomethylphenyl)-acetamid, N-(3-Methylaminomethylphenyl)-acetamid, 4-Aminomethyl-2-aminobenzolsulfonsäure, 4-Aminomethyl-2-acetylaminobenzolsulfonsäure, 4-Methylaminomethyl-2-acetylaminobenzolsulfonsäure, 5-Aminomethyl-2-aminobenzolsulfonsäure, 5-Aminomethyl-2-acetylaminobenzolsulfonsäure, 5-Methylaminomethyl-2-acetylaminobenzolsulfonsäure, Piperazin.

Die Bedingungen der Buchererreaktion sind an sich bekannt, sie sind z.B. in GB-A-230.457 beschrieben; sie erfolgt vorzugsweise in Wasser bei Temperaturen zwischen 80 °C und 130 °C, vorzugsweise zwischen 100 und 130 °C, wobei das Diamin bzw. Amid vorzugsweise im Überschuß eingesetzt wird.

Das Verfahren sei an folgenden Beispielen erläutert:

A)

23,9 g I-Säure, 14 g N,N'-Dimethylethylendiamin und 20,8 g Natriumhydrogensulfit werden bei pH 6 in 200 ml Wasser gelöst und 10 h auf 100 °C erhitzt. Das Produkt mit der Formel

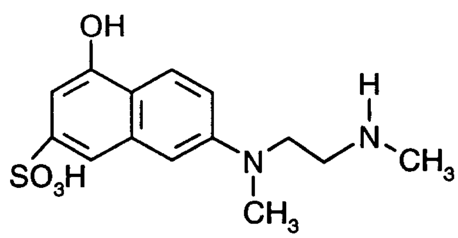

fällt beim Abkühlen aus, es wird isoliert, mit gesättigter Kochsalz-Lösung gewaschen und getrocknet.

Das NMR-Spektrum (250 MHz, Dimethylsulfoxid-$d_6$) zeigt folgende Signale: $\delta$ = 2.55 s, 3H; $\delta$ = 2.94 s, 3H; $\delta$ = 3.08 m, 2H; $\delta$= 3.4 m, 1H; $\delta$ = 3.66 m, 2H; $\delta$ = 6.88 d, 1H; $\delta$ = 6.94 d, 1H; $\delta$ = 7.12 q, 1H; $\delta$ = 7.47d, 1H; $\delta$ = 7.91 d, 1H;

$\delta$ = 9.8 breites s, 1H.

B)

23.9 g I-Säure, 16 g 1-Hydroxyethylamino-2-aminoethan und 20.8 g Natriumhydrogensulfit wurden bei pH 6 in 200 ml Wasser gelöst und 10 h auf 100 °C erhitzt. Das Produkt mit der Formel

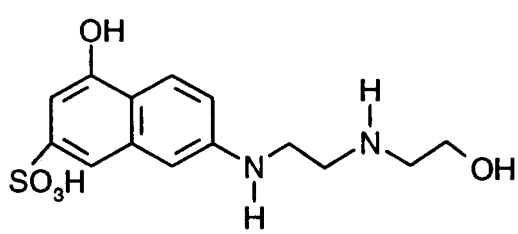

fällt beim Abkühlen aus, es wird isoliert, mit gesättigter Kochsalz-Lösung gewaschen und getrocknet.

Das NMR-Spektrum (250 MHz, Dimethylsulfoxid-$d_6$) zeigt folgende Signale:

$\delta$ = 3.09 m, 2H; $\delta$ = 3.14 m, 2H; $\delta$ = 3.44 m, 2H; $\delta$ = 3.68 m, 2H; $\delta$ = 5.29 breiter m, 1H; $\delta$ = 6.03 breiter t, 1H; $\delta$ = 6.82 s, 1H; $\delta$ = 6.9 m, 2H; $\delta$ = 7.45 s, 1H; $\delta$ = 7.96 d, 1H; $\delta$ = 8,5 breites m, 2H, $\delta$ = 9.9 breites s, 1H.

Das Verfahren zur Herstellung der Farbbasen der Formel (IX) ist an sich bekannt; es entspricht dem Verfahren zur Herstellung von C.I. Direct Blue 67.

Diazotierung und Kupplung erfolgen vorzugsweise in wässrigem Medium bei Temperaturen zwischen 0 °C und 30 °C, die alkalische Verseifung des Benzolsulfonsäureesters vorzugsweise bei Temperaturen zwischen 80 °C und 110 °C.

Bei dem erfindungsgemäßen Verfahren zur Herstellung von Farbstoffen der Formel (I) können Triazine vor oder nach der Kondensation mit der Farbbase mit Ammoniak, aliphatischen, aromatischen oder araliphatischen Aminen umgesetzt werden, welche noch einen Vinylsulfonrest oder eine durch Behandlung

mit Alkalien in einen solchen Rest überführbare Gruppe, wie eine 2-Sulfatoethylsulfonyl- oder eine 2-Chlorethylsulfonyl-Gruppe enthalten können oder mit aliphatischen, aromatischen oder araliphatischen Diaminen umgesetzt werden, die an einem Aminstickstoff mit einem Reaktivsystem aus der Reihe der Pyrimidine, wie sie z.B. oben aufgeführt sind, verbunden sind.

Solche Amine sind z.B. 2-Aminoethanol, 2-Methylaminoethanol, 2-Ethylaminoethanol, Anilin, N-Methylanilin, N-Ethylanilin, N-2-Hydroxyethylanilin, 2-Aminoethyl-2'-sulfatoethylsulfon, 3-Aminopropyl-2'-sulfatoethylsulfon, 2-(2'-Aminoethoxy-)ethyl-2''-sulfatoethylsulfon, 4-Aminobutyl-2'-sulfatoethylsulfon, 4-Aminophenyl-2'-sulfatoethylsulfon, 4-Methylaminophenyl-2'-sulfatoethylsulfon, 3-Aminophenyl-2'-sulfatoethylsulfon, 3-Methylaminophenyl-2'-sulfatoethylsulfon, 3-Aminobenzyl-2'-sulfatoethylsulfon, 4-Aminobenzyl-2'-sulfatoethylsulfon, 2-Phenylaminoethyl-2'-sulfatoethylsulfon, 3-Phenylaminoethyl-2'-sulfatoethylsulfon; solche Diamine sind z.B. die als Komponenten der Buchererreaktion oben angegebenen, gegebenenfalls nach Verseifung der Acetylamino-Gruppe.

Die Kondensationen der Amino-Gruppen der Ausgangskomponenten mit den Reaktivsystemen erfolgen unabhängig von der Reihenfolge in wäßrigem oder organisch-wäßrigen Medien in Anwesenheit von säurebindenden Mitteln. In Abhängigkeit von der Natur der Ausgangskomponenten erfolgt dabei die erste Stufe der Kondensation in pH-Bereichen von 2 bis 8, vorzugsweise 3 bis 7, und bei Temperaturen von 0 bis 60 °C, vorzugsweise von 0 bis 40 °C. Der Austausch des zweiten Halogenatoms des Triazins vollzieht sich im pH-Bereich von 4 bis 10, vorzugsweise von 5 bis 9, und im Temperaturbereich von 0 bis 60 °C, vorzugsweise 0 bis 30 °C.

Säurebindende Mittel sind beispielsweise Carbonate, Hydroxide oder Phosphate wie Natriumcarbonat, Natriumhydrogencarbonat, verdünnte Natronlauge, Di-oder Trinatriumphosphat oder Natriumfluorid.

Soll die Kondensation bzw. die Farbstoffsynthese direkt zu einer Farbstofflösung bzw. zu einer flüssigen Farbstoffpräparation führen, kann die Verwendung von Lithiumcarbonaten oder Lithiumhydroxid vorteilhaft sein, gegebenenfalls zusammen mit Lösungsvermittlern und/oder stabilisierenden Puffersystemen.

Die Reaktivfarbstoffe der Formel (I) können isoliert und zu trockenen Färbepräparationen verarbeitet werden. Die Isolierung erfolgt vorzugsweise bei möglichst niedrigen Temperaturen durch Aussalzen und Filtrieren. Die filtrierten Farbstoffe können gegebenenfalls nach Zugabe von Coupagemitteln und/oder Puffermitteln, z.B. nach Zugabe eines Gemisches gleicher Teile Mono- und Dinatriumphosphat getrocknet werden; vorzugsweise wird die Trocknung bei nicht zu hohen Temperaturen und unter vermindertem Druck vorgenommen. Durch Zerstäubungstrocknung des Herstellungsgemisches kann man in gewissen Fällen die erfindungsgemäßen trockenen Präparate direkt, d.h. ohne Zwischenisolierung der Farbstoffe, herstellen.

Die angegebenen Formeln sind die der freien Säuren. Bei der Herstellung werden im allgemeinen Salze erhalten, insbesondere die Alkalisalze wie Natrium-, Kalium- oder Lithiumsalze. Die Farbstoffe können auch als konzentrierte Lösungen eingesetzt werden.

Die erfindungsgemäßen Farbstoffe eignen sich hervorragend zum Farben und Bedrucken von natürlichen und synthetischen OH-oder amidgruppenhaltigen Materialien, insbesondere solchen aus Cellulose und Polyamiden. Sie sind besonders geeignet zum Färben von Cellulosematerialien im Auszieh- und Klotz-Kaltverweilfahren, sowie zum Bedrucken von Baumwolle und Zellwolle.

Man erhält bei gutem Aufbauvermögen und hohen Fixierausbeuten Färbungen mit guten Allgemeinechtheiten, insbesondere guten Naßechtheiten.

Erfindungsgemäß sind weiterhin Textilprodukte, enthaltend mit Farbstoffen der Formel (I) - mit Ausnahme der oben ausgenommenen Farbstoffe - gefärbte hydroxy- oder amidgruppenhaltige Materialien.

Beispiele

**Beispiel 1**

45,9 g H-Säure-O-Benzolsulfonsäureester werden in Wasser bei 0 °C mit 6,9 g Natriumnitrit diazotiert. Die dabei erhaltene Diazoniumverbindung wird bei pH 2,5 und 30 °C mit 13,7 g 2-Methoxy-5-methylanilin gekuppelt. Die Aminoazoverbindung wird bei 10 °C mit 6,9 g Natriumnitrit diazotiert. Die Diazoniumverbindung wird bei pH 7 und 20 °C mit 28,2 g 7-(2'-Aminoethylamino-)4-hydroxy-2-naphthalinsulfonsäure gekuppelt. Der Benzolsulfonylrest wird bei pH 12 und 90 °C abgespalten. Die Farbbase der Formel

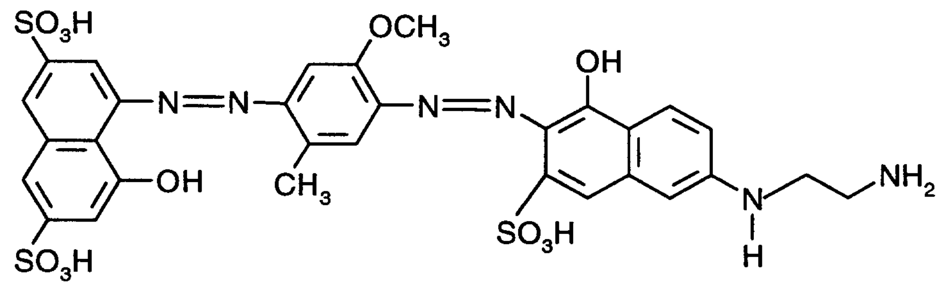

(a)

wird isoliert.

30,4 g 2-Amino-1,4-benzoldisulfonsäure werden in Wasser bei pH 4 und 0 °C mit 17,2 g 2,4,6-Trifluor-1,3,5-Triazin kondensiert. Die Lösung dieses Kondensationsproduktes wird zu einer Suspension der Farbbase in Wasser gegeben.

Die zweite Kondensation am Triazin erfolgt bei pH 7,5 und 40 °C. Der Farbstoff wird mit Kochsalz ausgefällt, isoliert und bei 40 °C im Vakuum getrocknet und besitzt die Formel

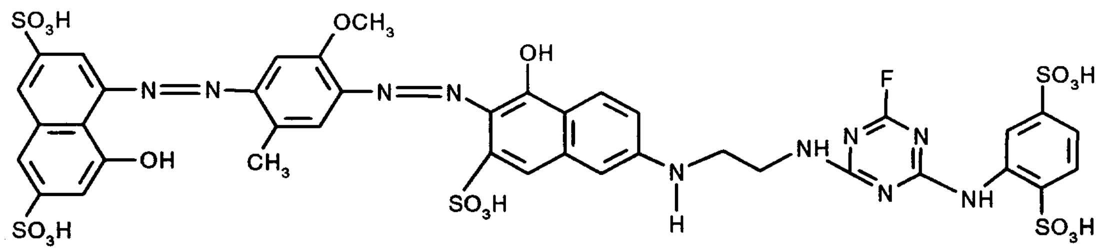

Er färbt Baumwolle und Viskose blau.

**Beispiel 2**

4,5 g H-Säure-O-Benzolsulfonsäureester werden in Wasser bei 0 °C mit 6,9 g Natriumnitrit diazotiert. Die Diazoniumverbindung wird bei pH 6 und 20 °C mit 22,3 g 8-Amino-2-naphthalinsulfonsäure gekuppelt. Die Aminoazoverbindung wird bei 20 °C mit 0,9 g Natriumnitrit diazotiert. Die Diazoniumverbindung wird bei pH 7 und 20 °C mit 32,6 g 7-[2-(2'-Hydroxyethylamino)-ethylamino]-4-hydroxy-2-naphthalinsulfonsäure gekuppelt. Der Benzolsulfonylrest wird bei pH 12 und 50 °C abgespalten. Die Farbbase der Formel

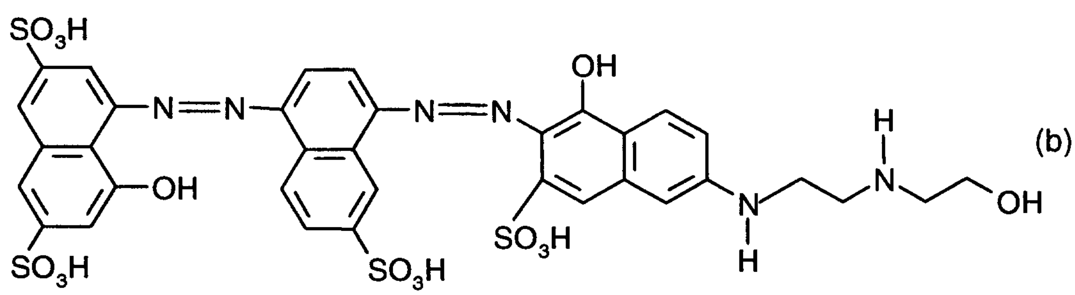

(b)

wird isoliert, wieder in Wasser suspendiert und mit 18 g 5-Chlor-4,6-difluorpyrimidin bei pH 7,5 und 40 °C kondensiert. Der Farbstoff wird mit Kochsalz ausgefällt, isoliert und bei 40 °C im Vakuum getrocknet und hat die Formel

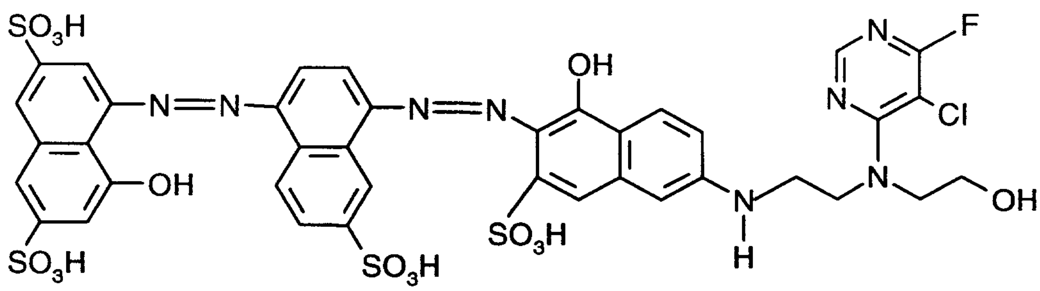

Er färbt Baumwolle und Viskose blau.

**Beispiel 3**

45,9 g H-Säure-O-Benzolsulfonsäureester werden in Wasser bei 0 °C mit 6,9 g Natriumnitrit diazotiert. Die dabei erhaltene Diazoniumverbindung wird bei pH 6 und 20 °C mit 22,3 g 5-Amino-2-naphthalinsulfonsäure gekuppelt. Die Aminoazoverbindung wird bei 20 °C mit 6,9 g Natriumnitrit diazotiert. Die Diazoniumverbindung wird bei pH 7 und 20 °C mit 30,8 g 7-(1-Piperazinyl-)4-hydroxy-2-naphthalinsulfonsäure gekuppelt. Der Benzolsulfonylrest wird bei pH 12 und 50 °C abgespalten. Die Farbbase der Formel

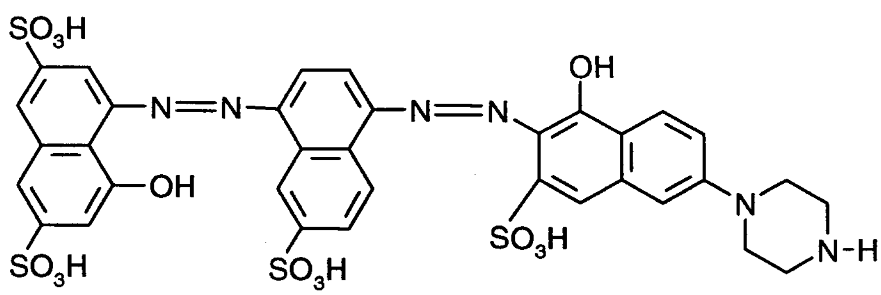

(c)

wird isoliert.

38,7 g 2-Phenylaminoethyl-2'-sulfatoethylsulfon werden im Wasser bei pH 7 und 0 °C mit 22,2 g 2,4,6-Trichlor-1,3,5-triazin kondensiert. Die Lösung dieses Kondensationsproduktes wird zu einer Suspension der Farbbase in Wasser gegeben. Die zweite Kondensation am Triazin erfolgt bei pH 7,5 und 40 °C. Der Farbstoff wird mit Kochsalz ausgefällt, isoliert und bei 40 °C im Vakuum getrocknet und besitzt die Formel

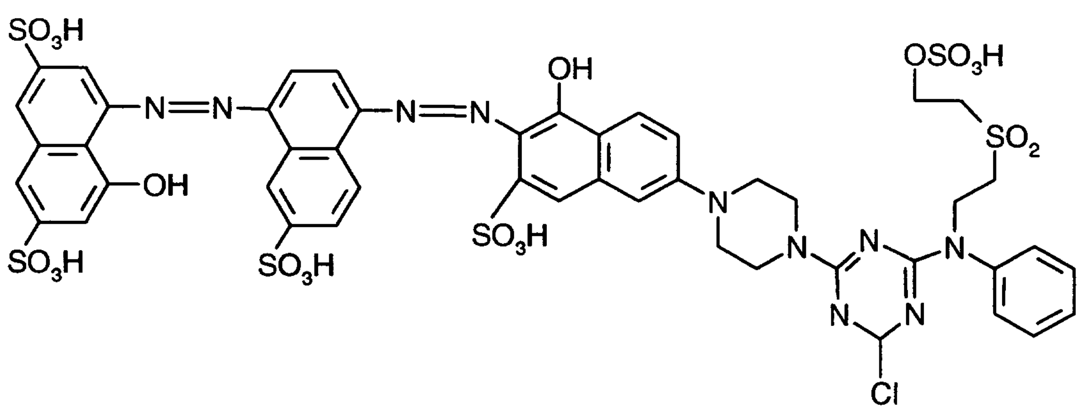

Er färbt Baumwolle und Viskose blau.

In der folgenden Tabelle sind weitere Farbstoffe aufgeführt, die Baumwolle und Viskose blau färben.

Die Umsetzung der Farbbase mit dem Reaktivsystem erfolgt bei pH 7 und 40 °C.

Tabelle 1

| Bsp.-Nr. | 1. Diazonium-verbindung | 1. Kupplungs-komponente | 2. Kupplungs-komponente | Reaktivsystem |
|---|---|---|---|---|
| | | | | |
| 4 | 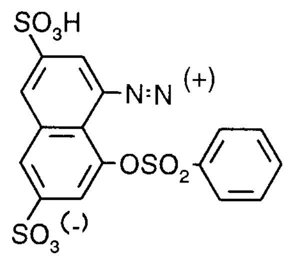 | 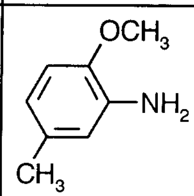 | 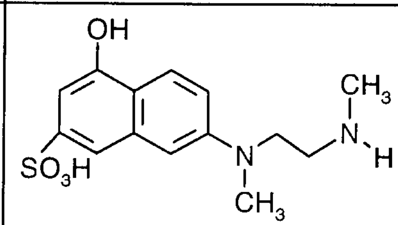 | 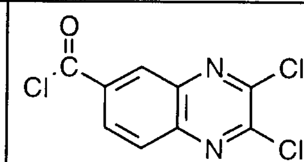 |
| 5 | 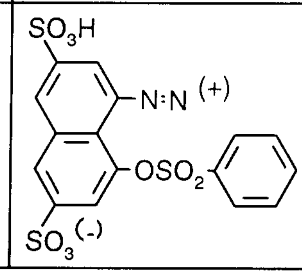 | 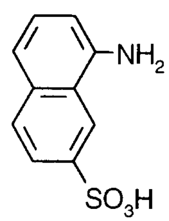 | 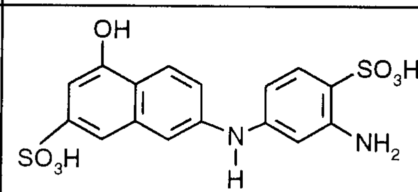 | 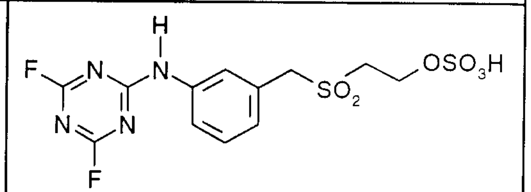 |

**Tabelle 1** (Fortsetzung)

| Bsp.-Nr. | 1. Diazonium-verbindung | 1. Kupplungs-komponente | 2. Kupplungs-komponente | Reaktivsystem |
|---|---|---|---|---|
| | | | | |
| 6 | 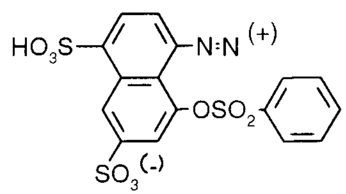 | 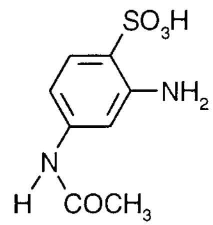 | 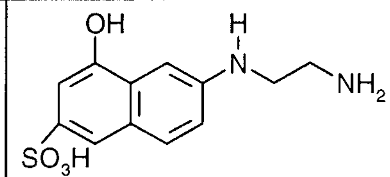 | 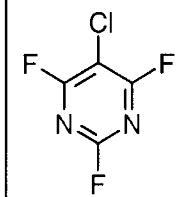 |
| 7 | 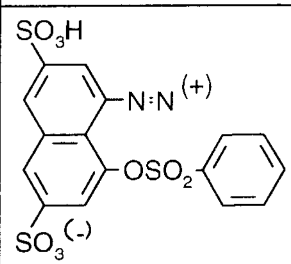 | 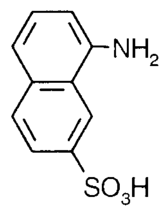 | 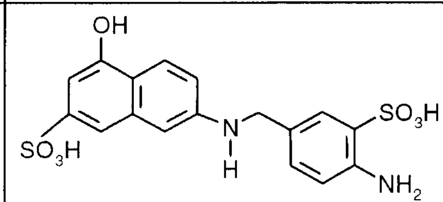 | 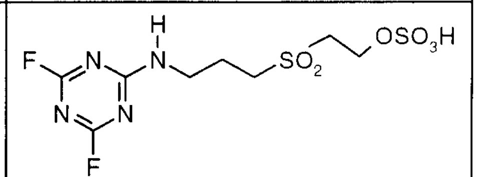 |

**Tabelle 1** (Fortsetzung)

| Bsp.-Nr. | 1. Diazonium-verbindung | 1. Kupplungs-komponente | 2. Kupplungs-komponente | Reaktivsystem |
|---|---|---|---|---|
| | | | | |
| 8 | 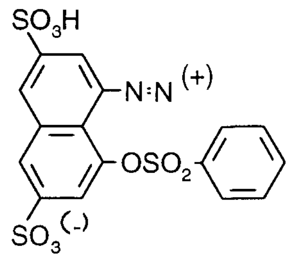 | 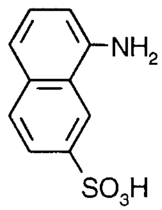 | 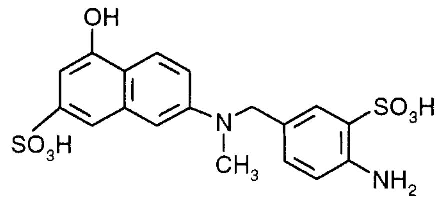 | 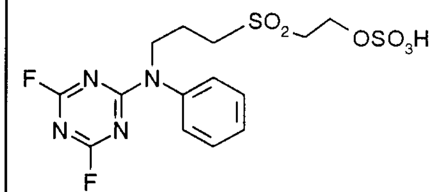 |
| 9 | 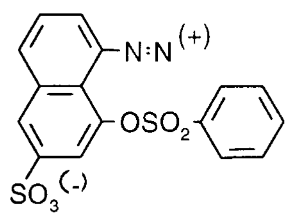 | 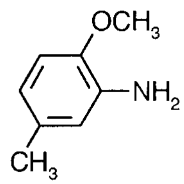 | 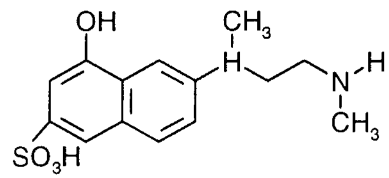 | 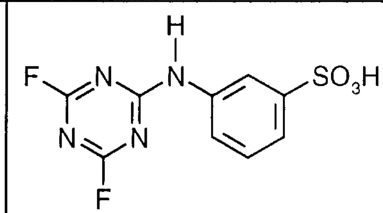 |
| 10 | 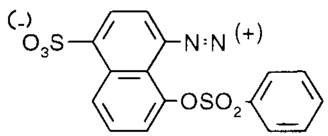 | 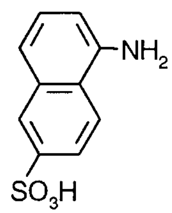 | 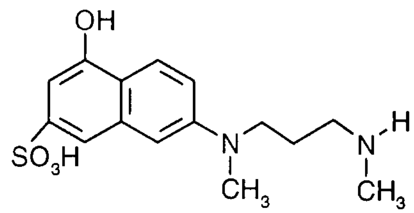 | 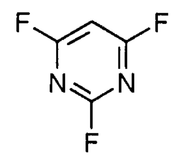 |

**Tabelle 1** (Fortsetzung)

| Bsp.-Nr. | 1. Diazonium-verbindung | 1. Kupplungs-komponente | 2. Kupplungs-komponente | Reaktivsystem |
|---|---|---|---|---|
| 11 | 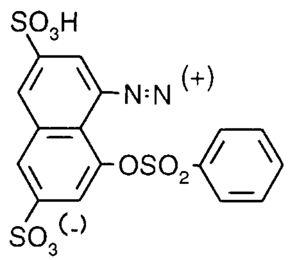 | 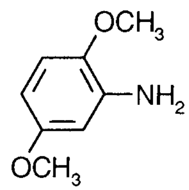 | 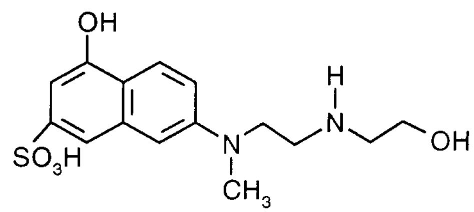 | 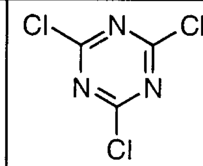 |
| 12 | 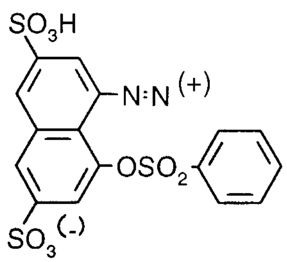 | 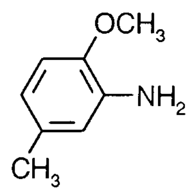 | 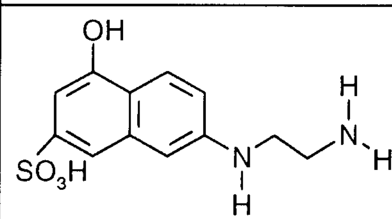 | 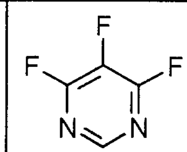 |
| 13 | 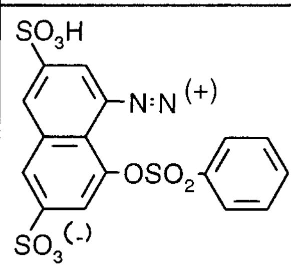 | 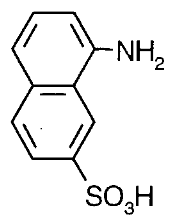 | 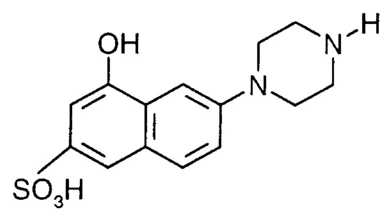 | 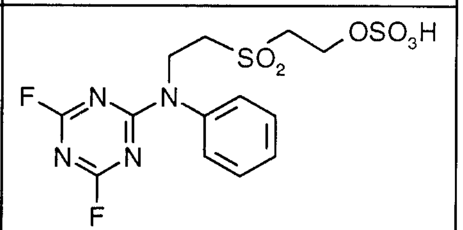 |

**Tabelle 1** (Fortsetzung)

| Bsp.-Nr. | 1. Diazoniumverbindung | 1. Kupplungskomponente | 2. Kupplungskomponente | Reaktivsystem |
|---|---|---|---|---|
| 14 | 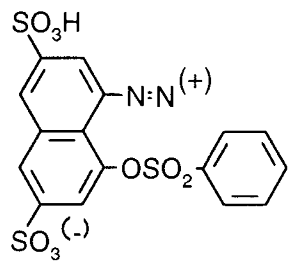 | 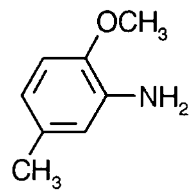 | 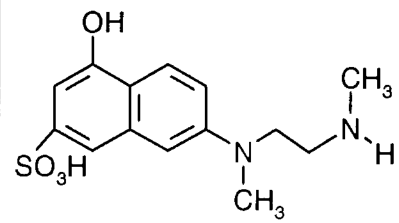 | 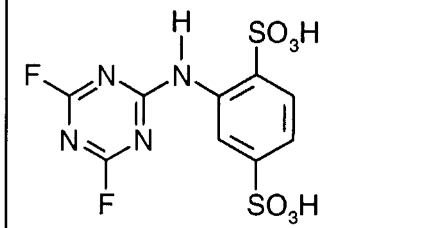 |
| 15 | 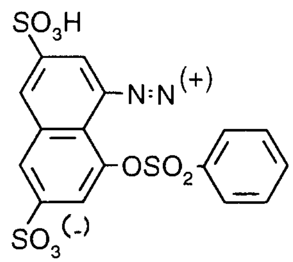 | 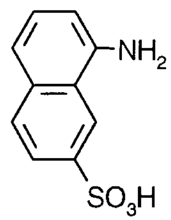 | 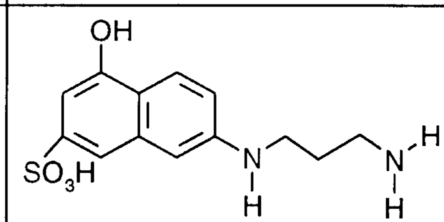 | 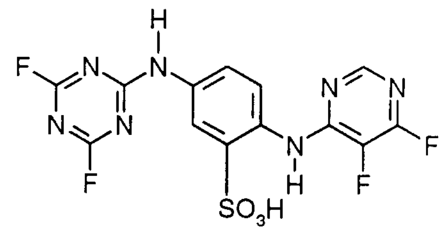 |
| 16 | 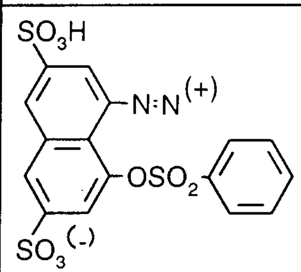 | 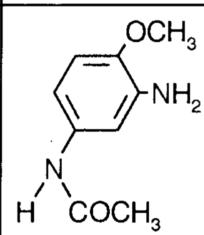 | 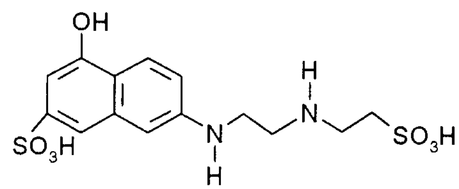 | 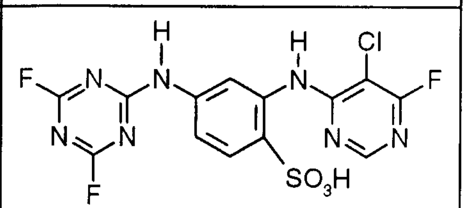 |

**Tabelle 1** (Fortsetzung)

| Bsp.-Nr. | 1. Diazonium-verbindung | 1. Kupplungs-komponente | 2. Kupplungs-komponente | Reaktivsystem |
|---|---|---|---|---|
| 17 | 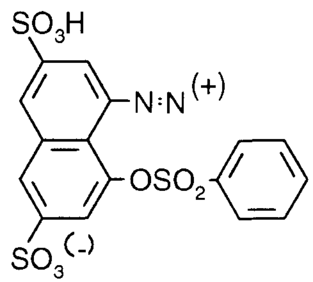 | 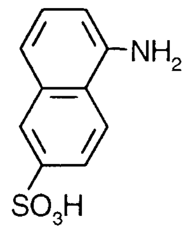 | 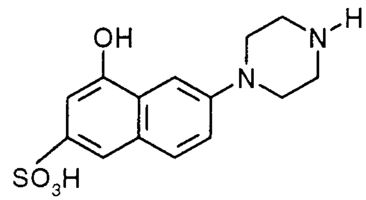 | 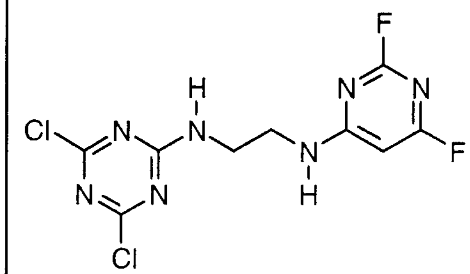 |
| 18 | 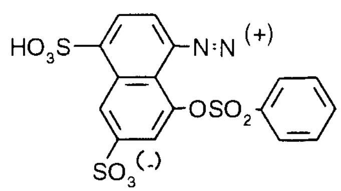 | 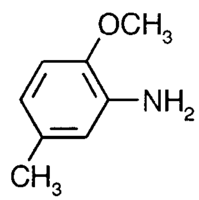 | 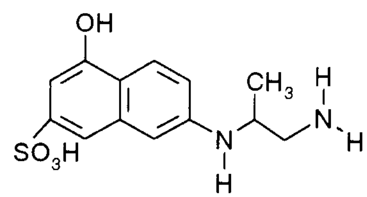 | 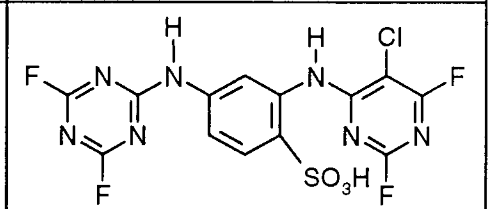 |
| 19 | 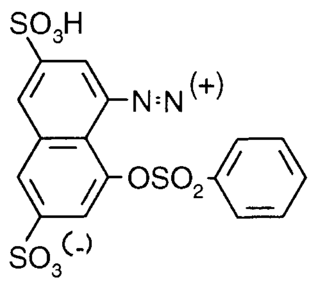 | 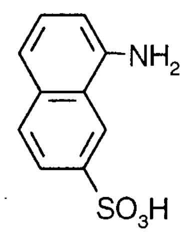 | 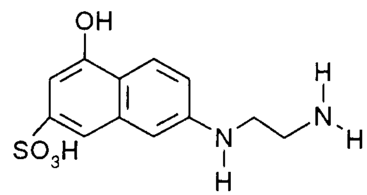 | 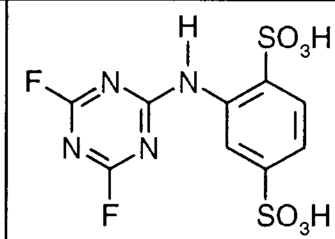 |

**Tabelle 1** (Fortsetzung)

| Bsp.-Nr. | 1. Diazonium-verbindung | 1. Kupplungs-komponente | 2. Kupplungs-komponente | Reaktivsystem |
|---|---|---|---|---|
| 20 | 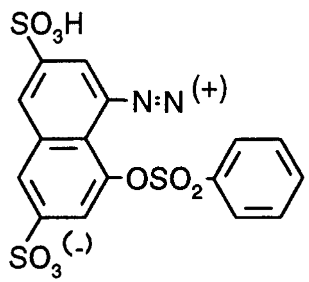 | 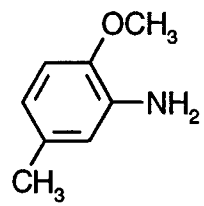 | 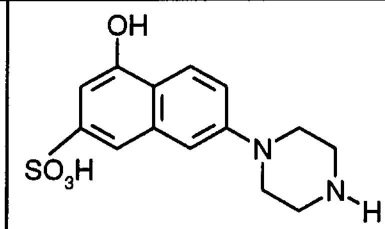 | 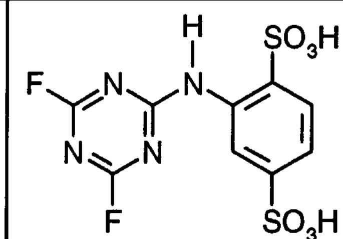 |
| 21 | 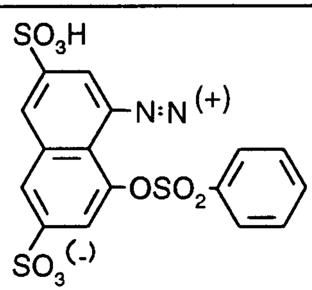 | 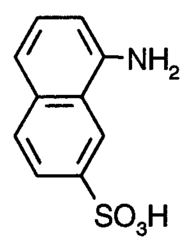 | 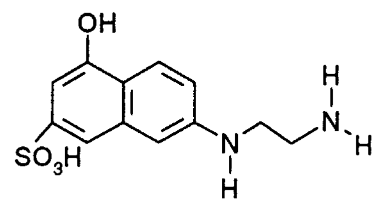 | 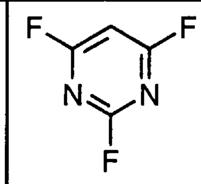 |
| 22 | 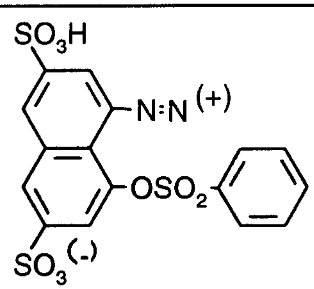 | 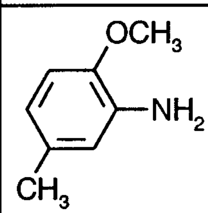 | 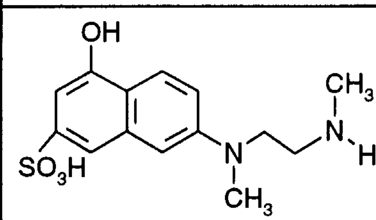 | 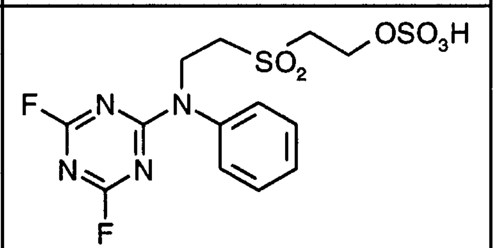 |

**Tabelle 1** (Fortsetzung)

| Bsp.-Nr. | 1. Diazonium-verbindung | 1. Kupplungs-komponente | 2. Kupplungs-komponente | Reaktivsystem |
|---|---|---|---|---|
| 23 | 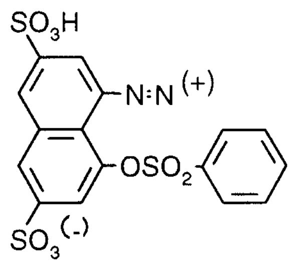 | 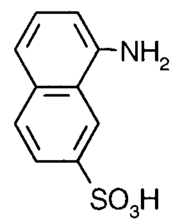 | 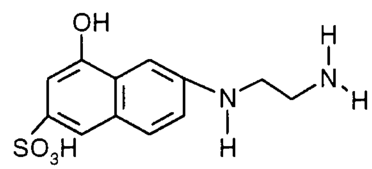 | 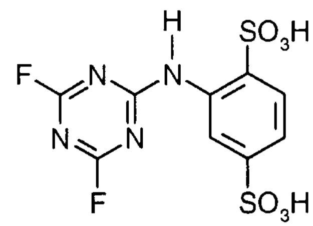 |
| 24 | 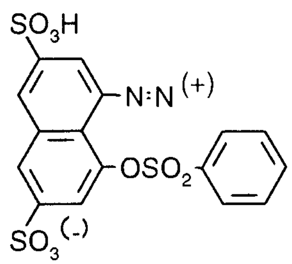 | 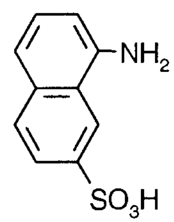 | 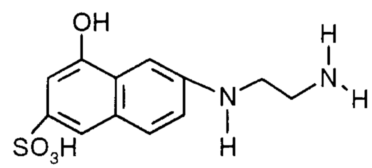 | 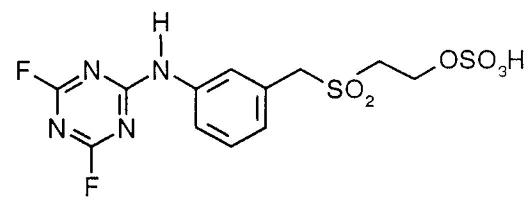 |
| 25 | 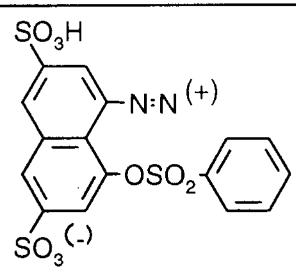 | 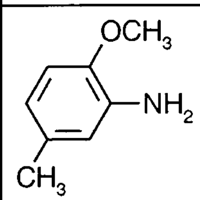 | 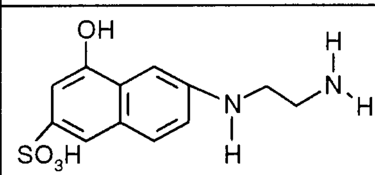 | 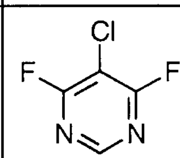 |

**Tabelle 1** (Fortsetzung)

| Bsp.-Nr. | 1. Diazoniumverbindung | 1. Kupplungskomponente | 2. Kupplungskomponente | Reaktivsystem |
|---|---|---|---|---|
| 26 | 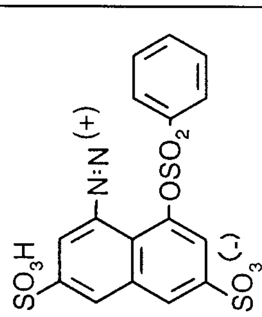 | 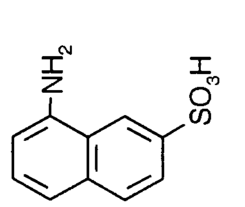 | 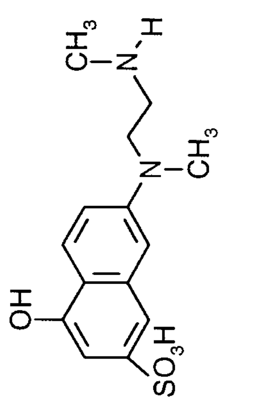 | 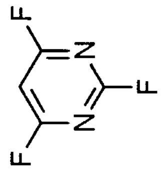 |
| 27 | 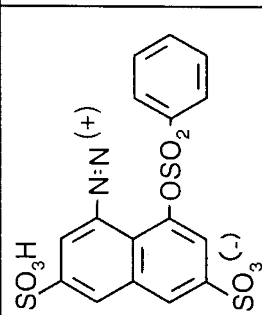 | 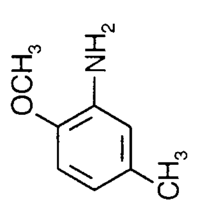 | 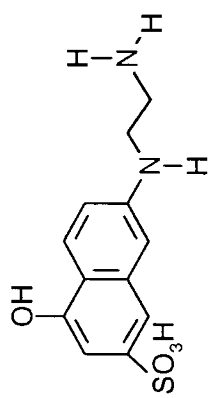 | 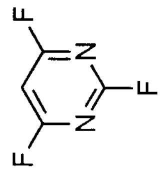 |
| 28 | 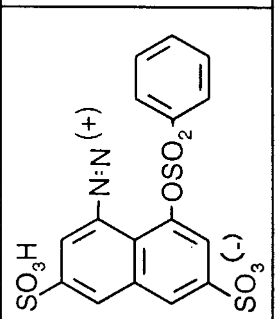 | 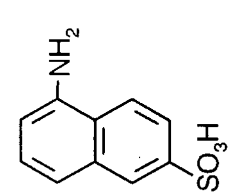 | 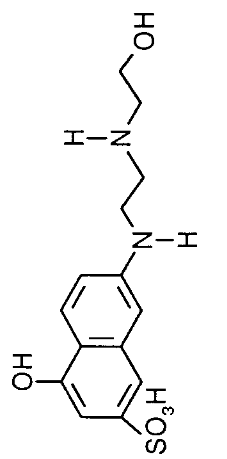 | 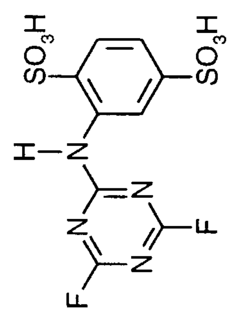 |

## Beispiel 29

89 g der Farbbase aus Beispiel 2 (Formel b) werden in Wasser gelöst und bei pH 7 und 0 ° C mit 18,5 g Cyanurchlorid kondensiert. Zu der dabei erhaltenen Lösung werden 38,7 g 2-Phenylaminoethyl-2'-sulfatoethylsulfon zugesetzt; die zweite Kondensation am Triazin erfolgt bei pH 7,5 und 40 ° C. Der Farbstoff wird mit Kochsalz ausgefällt, isoliert und bei 40 ° C im Vakuum getrocknet und besitzt die Formel

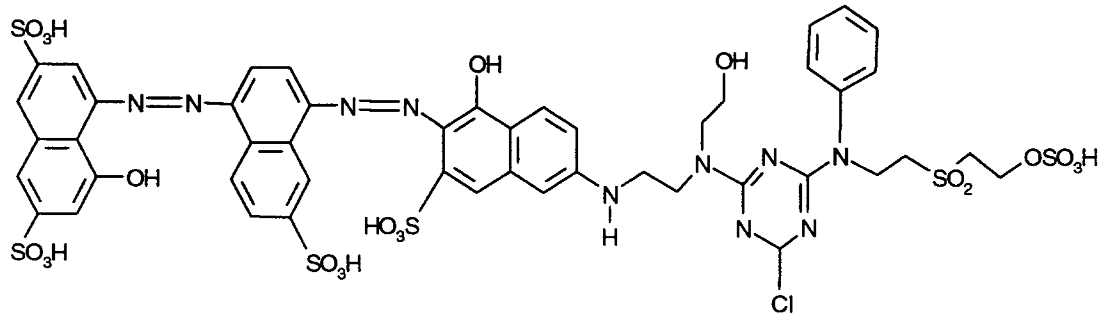

Er färbt Baumwolle und Viskose blau.

**Patentansprüche**

1. Reaktivfarbstoffe, die in Form der freien Säure der Formel (I) entsprechen,

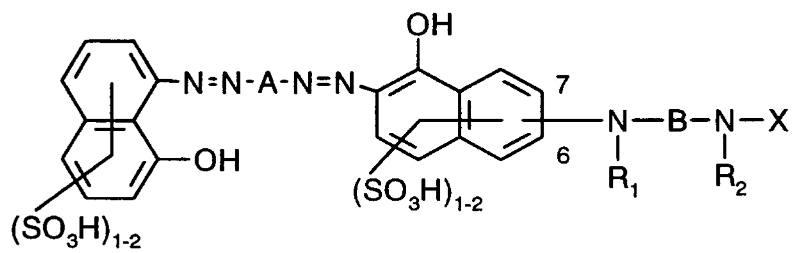

(I)

in der der Rest

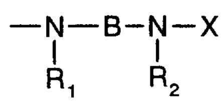

an die 6- oder 7-Position des Naphthalinssystems gebunden ist, worin

A einen Rest der Formel

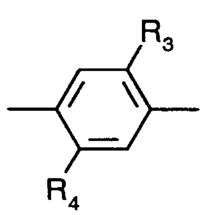

oder

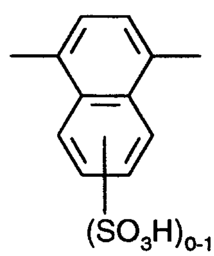

B ein Brückenglied,

$R_1$ und $R_2$ unabhängig voneinander H, gegebenenfalls substituiertes $C_1$-$C_3$-Alkyl oder gegebenenfalls substituiertes Phenyl bedeutet oder

| | |
|---|---|
| B, $R_1$ und $R_2$ | zusammen mit den beiden Stickstoffatomen ein gegebenenfalls substituiertes Piperazin bilden, |
| $R_3$ | H, $C_1$-$C_4$-Alkyl, $C_1$-$C_4$-Alkoxy oder $SO_3H$, |
| $R_4$ | H, $C_1$-$C_4$-Alkyl, $C_1$-$C_4$-Alkoxy, $NHCOCH_3$, $NHCONH_2$ oder $NHCOCH_2OH$ bedeutet und |
| X | ein Reaktivsystem aus der Reihe der 2,3-Dichlorchinoxaline, der Pyrimidine oder der Triazine darstellt, wobei Reaktivfarbstoffe der Formel (I) ausgenommen sind, worin |
| B | eine sulfogruppenhaltige Phenylgruppe und |
| X | einen 2-Chlor-4-amino-1,3,5-Triazinrest oder einen 2-Chlor-4-sulfoanilino-1,3,5-Triazinrest bedeutet. |

**2.** Reaktivfarbstoffe gemäß Anspruch 1, dadurch gekennzeichnet, daß
X für einen Triazinrest steht, der mit einem Amin substituiert ist, welches gegebenenfalls eine Vinylsulfonyl- oder eine unter alkalischen Bedingungen in diese überführbare Gruppe enthält oder der mit einem Diamin substituiert ist, welches gegebenenfalls an dem Aminstickstoff, das nicht mit dem Triazinrest verknüpft ist, mit weiteren Reaktivsystemen aus der Reihe der Pyrimidine substituiert ist.

**3.** Reaktivfarbstoffe gemäß Anspruch 1, dadurch gekennzeichnet, daß sie in der Form der freien Säure der Formel (II) entsprechen,

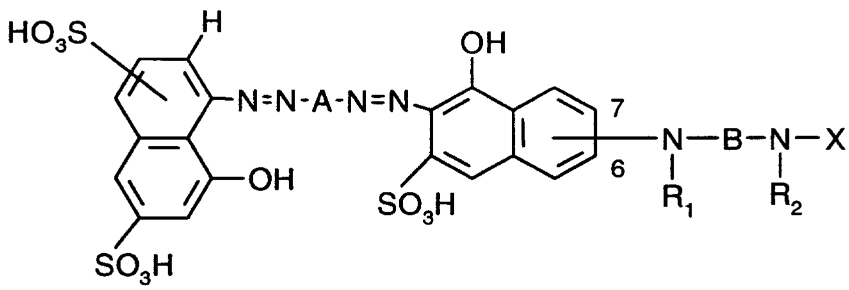

(II)

worin

A einen Rest der Formel

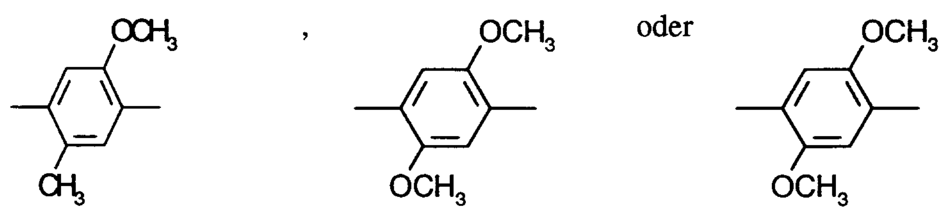

| | |
|---|---|
| $R_1$ | H, $CH_3$ oder $C_2H_4OH$, |
| $R_2$ | H, $CH_3$, $C_2H_4OH$, $C_2H_4SO_3H$, $C_6H_5$ oder $C_6H_4SO_3H$ bedeutet oder |
| $R_1$ und $R_2$ | zusammen mit B und den beiden N-Atomen ein Piperazin bilden, |
| B | geradkettiges oder verzweigtes $C_2$-$C_6$-Alkylen, $C_5$-$C_6$-Cycloalkylen, gegebenenfalls mit einer Sulfogruppe substituiertes Phenylen, ein Rest der Formel -$C_2H_4$-O-$C_2H_4$- oder einen Rest der Formel |

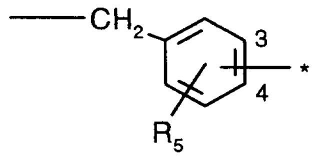

bedeutet, worin die Gruppe

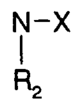

über die mit * markierte Bindung mit der 3- oder 4-Position des Ringes verknüpft ist, und

$R_5$ für H oder $SO_3H$ steht.

**4.** Reaktivfarbstoffe gemäß Anspruch 1, dadurch gekennzeichnet, daß sie in Form ihrer freien Säure der Formel (III) entsprechen,

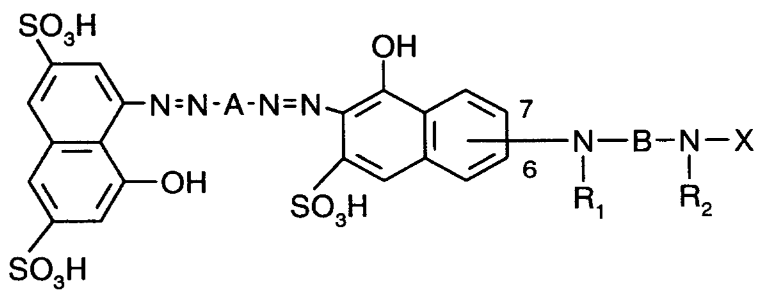 (III)

worin

B $C_2H_4$, $C_3H_6$, $C_4H_8$ oder einen Rest der Formel

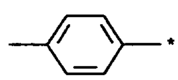 , 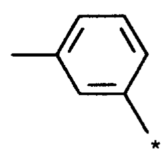 , 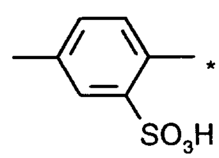 ,

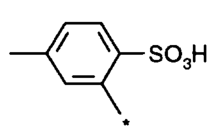 , 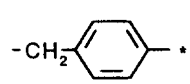 , 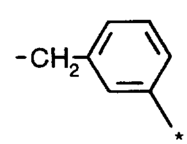 ,

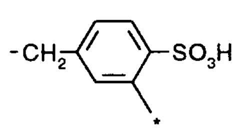 oder 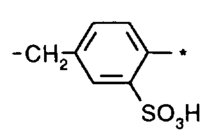

bedeutet, wobei die Gruppe

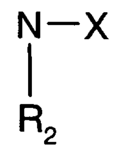

über die mit * markierte Bindung verknüpft ist,

X einen Rest der allgemeinen Formel

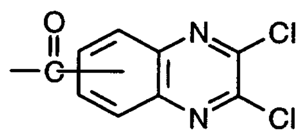 , 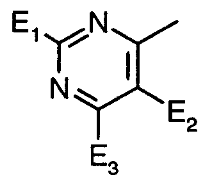 , 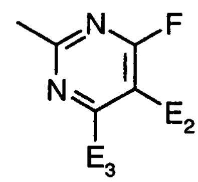 ,

(IV) (Va) (Vb)

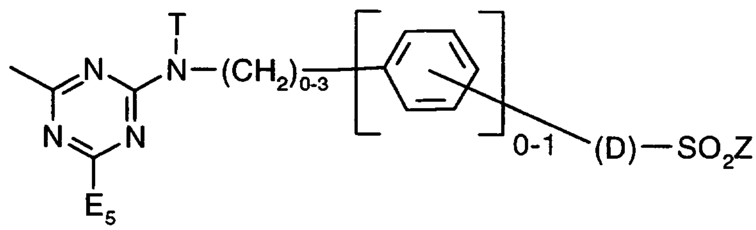

(VI) ,

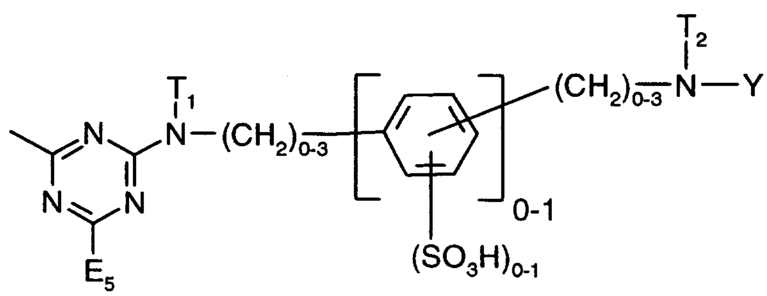

(VII)

oder

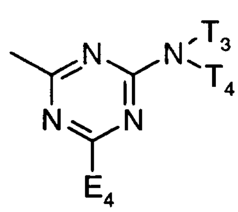

(VIII)

bedeutet,

worin

| | |
|---|---|
| $E_1$ | für H oder F, |
| $E_2$ | für H, Cl oder CN, |
| $E_3$ | für F oder $CH_3$, |
| $E_4$, $E_5$ | für F oder Cl steht, |
| T, $T_1$ | H, gegebenenfalls substituiertes $C_1$-$C_4$-Alkyl oder gegebenenfalls substituiertes Phenyl, |
| $T_2$ | H oder gegebenenfalls substituiertes $C_1$-$C_4$-Alkyl, |

T3 H, gegebenenfalls substituiertes $C_1$-$C_4$-Alkyl oder gegebenenfalls sulfonsäuregruppenhaltiges Phenyl
T4 H oder gegebenenfalls substituiertes $C_1$-$C_4$-Alkyl,
D einen Rest der Formel $-(CH_2)_{1-6}-$ oder $-C_2H_4-O-C_2H_4-$,
Y einen Rest der Formeln (Va) oder (Vb),
Z $-CH=CH_2$ oder eine unter alkalischen Bedingungen in Vinylsulfonyl überführbare Gruppe bedeutet.

**5.** Reaktivfarbstoffe der Formel (III) gemäß Anspruch 4, dadurch gekennzeichnet, daß
B für $C_2H_4$, $C_3H_6$, $C_4H_8$ oder einen Rest der Formel

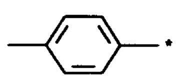 , 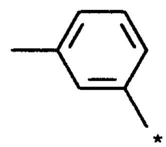 , 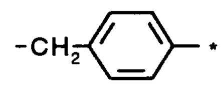 ,

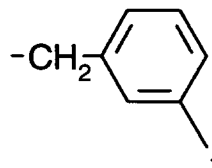 , 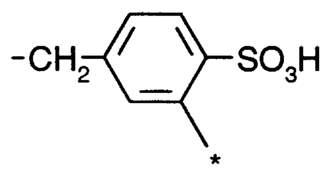 oder 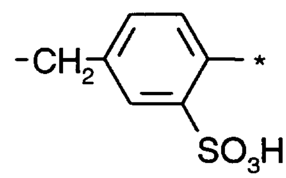

steht, wobei die Gruppe

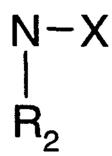

über die mit * markierte Bindung verknüpft ist.

**6.** Verfahren zur Herstellung von Reaktivfarbstoffen der Formel (I)

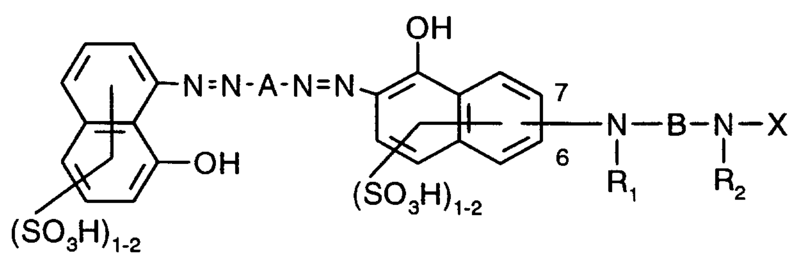 (I)

gemäß Anspruch 1, dadurch gekennzeichnet, daß Farbbasen der allgemeinen Formel (IX)

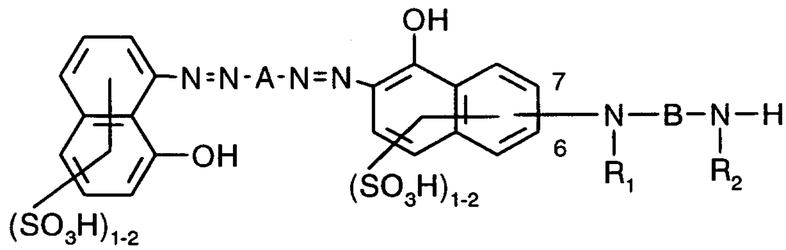

(IX)

mit Reaktivsystemen der Formel

Hal - X

umgesetzt werden, worin

X, A, B, $R_1$ und $R_2$ die in Anspruch 1 angegebene Bedeutung besitzen und
Hal für Halogen steht.

**7.** Verbindungen der Formel (IX)

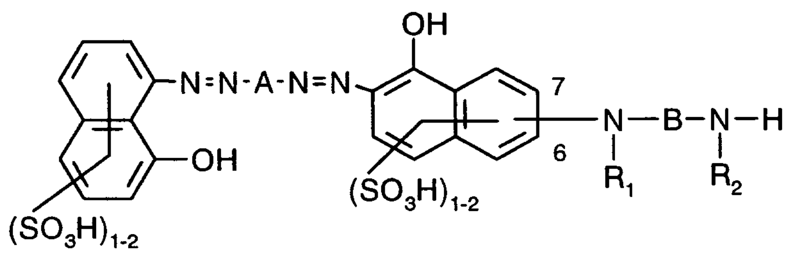

(IX)

worin

$R_1$ H, $CH_3$ oder $C_2H_4OH$,
$R_2$ H, $CH_3$, $C_2H_4OH$, $C_2H_4SO_3H$, $C_6H_5$ oder $C_6H_4SO_3H$,
B geradkettiges oder verzweigtes $C_2$-$C_6$-Alkylen, $C_5$-$C_6$-Cycloalkylen, einen Rest der Formel $-C_2H_4-O-C_2H_4-$ oder einen Rest der Formel

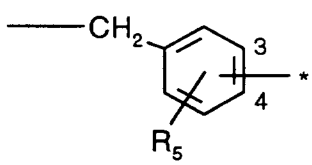

bedeutet, worin die Gruppe

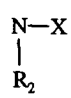

über die mit * markierte Bindung mit der 3- oder 4-Position des Ringes verknüpft ist,
$R_5$ für H oder $SO_3H$ steht und
A die in Anspruch 1 genannte Bedeutung hat.

**8.** Verfahren zur Herstellung von Verbindungen der Formel (IX) gemäß. Anspruch 7, dadurch gekennzeichnet, daß man Diazoniumverbindungen der Formel

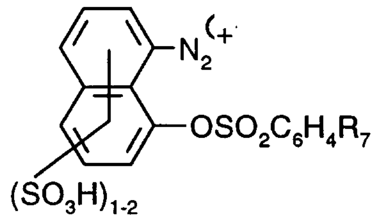

(X)

auf Kupplungskomponenten der allgemeinen Formel

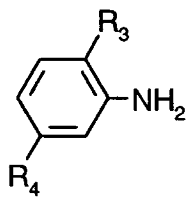

oder

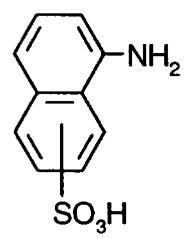

(XIa) (XIb)

kuppelt, worin $R_3$ und $R_4$ die in Anspruch 1 angegebene Bedeutung haben, die so erhaltenen Aminoazoverbindungen ihrerseits diazotiert und diese Diazoniumazoverbindungen mit Kupplungskomponenten der allgemeinen Formel

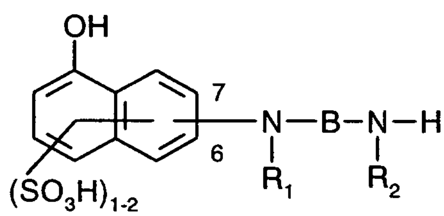

(XII)

kuppelt und das erhaltene Disazoprodukt der Formel (XIII)

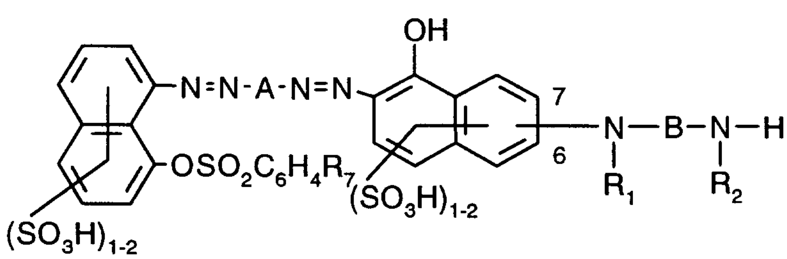

(XIII),

worin

| | |
|---|---|
| $R_7$ | H, $C_1$-$C_4$-Alkyl oder Halogen bedeutet, |
| B, $R_1$ und $R_2$ | die in Anspruch 7 angegebene Bedeutung haben, |

verseift.

**9.** Verbindungen der Formel (XIII)

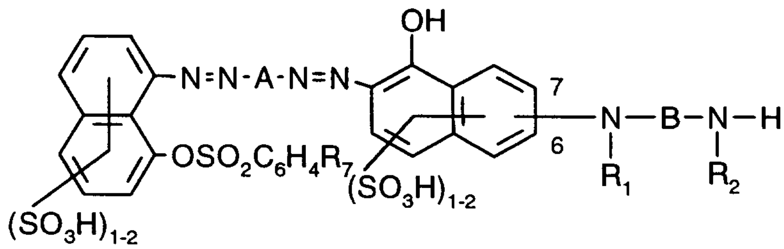

(XIII),

worin

| | |
|---|---|
| $R_7$ | H, $C_1$-$C_4$-Alkyl oder Halogen bedeutet, und |
| A, B, $R_1$ und $R_2$ | die in Anspruch 7 angegebene Bedeutung haben. |

**10.** Verbindungen der Formel (XII)

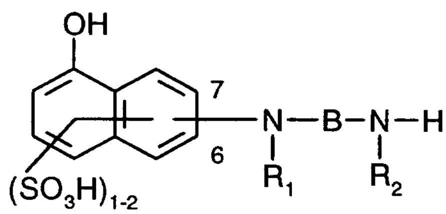

(XII),

worin

$R_1$ H, $CH_3$ oder $C_2H_4OH$,

$R_2$ H, $CH_3$, $C_2H_4OH$, $C_2H_4SO_3H$, $C_6H_5$ oder $C_6H_4SO_3H$ bedeutet,

B geradkettiges oder verzweigtes $C_2$-$C_6$-Alkylen, $C_5$-$C_6$-Cycloalkylen, einen Rest der Formel $-C_2H_4$-O-$C_2H_4$-oder einen Rest der Formel

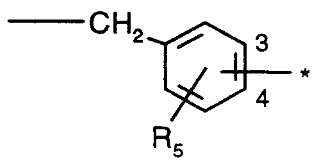

bedeutet, worin die Gruppe

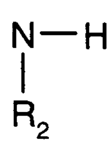

über die mit * markierte Bindung mit der 3- oder 4-Position des Rings verknüpft ist und

$R_5$ für H oder $SO_3H$ steht,

mit der Maßgabe, daß $R_2$ ≠ Wasserstoff, wenn B für Ethylen und $R_1$ für Wasserstoff steht.

**11.** Verfahren zur Herstellung von Verbindungen der Formel (XII) gemäß Anspruch 10

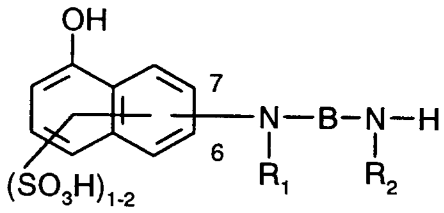 (XII),

dadurch gekennzeichnet, daß man in einer Buchereaktion die entsprechenden Aminohydroxynaphthalinsulfonsäuren oder Dihydroxynaphthalinsulfonsäuren der allgemeinen Formel

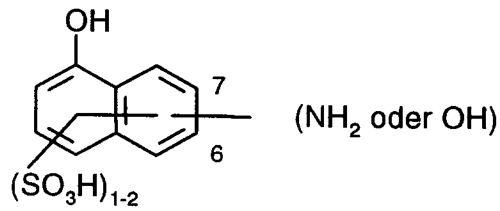

mit Diaminen der allgemeinen Formel

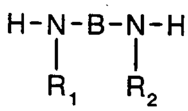 (XIV )

oder Monocarbonsäureamiden der allgemeinen Formel

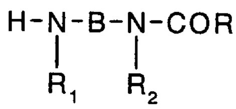 (XIVa ),

worin

R ein Alkyl- oder Arylrest ist und B, $R_1$ und $R_6$ die in Anspruch 10 genannte Bedeutung besitzen,

umsetzt.

**12.** Verwendung der Reaktivfarbstoffe gemäß Anspruch 1 zum Färben oder Bedrucken von natürlichen oder synthetischen hydroxyl- oder amidgruppenhaltigen Materialien.

**13.** Textilprodukte, enthaltend mit Farbstoffen gemäß Anspruch 1 gefärbte hydroxy- oder amidgruppenhaltige Materialien.

Europäisches Patentamt

# EUROPÄISCHER RECHERCHENBERICHT

Nummer der Anmeldung
EP 94 10 5981

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.5) |
|---|---|---|---|
| X,D | DE-B-11 51 625 (CIBA AG.)<br>* Spalte 3, Zeile 57 - Spalte 4, Zeile 9; Ansprüche 1,5; Beispiel 2 *<br>* Spalte 5, Zeile 31 - Zeile 35 *<br>--- | 1-13 | C09B62/03<br>C09B62/09<br>C09B62/25<br>C09B62/41<br>C09B62/513<br>C09B31/08<br>C07C309/50<br>D06P3/24<br>D06P3/66 |
| A | EP-A-0 534 903 (CIBA-GEIGY AG.)<br>* Beispiel 24 *<br>--- | 7,8 | |
| A | CHEMICAL ABSTRACTS, vol. 115, no. 2, 15. Juli 1991, Columbus, Ohio, US; abstract no. 10841g,<br>J.BARANIAK ET AL. 'Manufacture of C.I. Direct Blue 67'<br>Seite 108 ;<br>* Zusammenfassung *<br>& PL-A-148 354 (ORGANIKA)<br>----- | 7-11 | |

RECHERCHIERTE SACHGEBIETE (Int.Cl.5)
C09B
C07C

Der vorliegende Recherchenbericht wurde für alle Patentansprüche erstellt

| Recherchenort | Abschlußdatum der Recherche | Prüfer |
|---|---|---|
| DEN HAAG | 16. August 1994 | Ginoux, C |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument